# EUROPEAN PATENT SPECIFICATION

(11) **EP 1 637 167 B1**
(45) Date of publication and mention of the grant of the patent: **12.02.2014**
(21) Application number: 05108494.5
(22) Date of filing: 15.09.2005
(51) Int. Cl.: A61K 47/48, A61K 31/57

(54) **Injectable formulations containing a complex between progesterone and hydroxypropyl-beta-cyclodextrin**
Einspritzbare Formulierungen mit einem Komplex zwischen Hydroxypropyl-beta-Cyclodextrin und Progesteron
Formulations injectables contenant un complexe entre la progesterone et l'hydroxypropyl-bêta-cyclodextrine

(30) Priority: 16.09.2004 IT MI20041763
(43) Date of publication of application: 22.03.2006
(73) Proprietor: ALTERGON S.A., CH-6903 Lugano (CH)
(72) Inventor: Zoppetti, Giorgio, 20155, MILANO (IT); Pizzutti, Marco, 21046, MALNATE (IT); Puppini, Nadia, 22100, COMO (IT)
(74) Representative: Gerli, Paolo

(56) References cited:
- WO-A-85/02767
- CN-A- 1 517 091
- US-A- 4 727 064
- GOSS C W ET AL: "Behavioral effects and anatomic correlates after brain injury: A progesterone dose-response study" PHARMACOLOGY BIOCHEMISTRY AND BEHAVIOR 2003 UNITED STATES, vol. 76, no. 2, 2003, pages 231-242, XP002362320 ISSN: 0091-3057
- DATABASE WPI Section Ch, Week 200477 Derwent Publications Ltd., London, GB; Class B01, AN 2004-776335 XP002362431 & CN 1 517 091 A (DONG Y) 4 August 2004 (2004-08-04)
- CERCHIARA T ET AL: "Effect of chitosan on progesterone release from hydroxypropyl-[beta]-cyclodextrin complexes" INTERNATIONAL JOURNAL OF PHARMACEUTICS 04 JUN 2003 NETHERLANDS, vol. 258, no. 1-2, 4 June 2003 (2003-06-04), pages 209-215, XP002362321 ISSN: 0378-5173
- BREWSTER M E ET AL: "THE POTENTIAL USE OF CYCLODEXTRINS IN PARENTERAL FORMULATIONS" JOURNAL OF PARENTERAL SCIENCE AND TECHNOLOGY, vol. 43, no. 5, September 1989 (1989-09), pages 231-240, XP001002818 ISSN: 0279-7976
- SZENTE L ET AL: "Highly soluble cyclodextrin derivatives: chemistry, properties, and trends in development" ADVANCED DRUG DELIVERY REVIEWS, AMSTERDAM, NL, vol. 36, 1999, pages 17-28, XP002231320 ISSN: 0169-409X
- RDI DIVISION OF FITZGERALD INDUSTRIES INTL: "Cyclodextrins"[Online] 1 October 2004 (2004-10-01), XP002362395 Retrieved from the Internet: URL:http://www.researchd.com/janssen/41020 0.htm> [retrieved on 2006-01-06]
- "Hydroxypropylbetadex" In: "European Pharmacopeia supplement 4.6 to the Fourth Edition", 1 June 2003 (2003-06-01), Directorate for the Quality of Medicines of the Council of Europe; ISBN: 92-871-5102-4 page pages 4036-4037,
- 'Material Safety Data Sheet for Encapsin (HPB Cyclodextrin) produced/distributed by American Maize-Products Company', [Online] Retrieved from the Internet: <URL:http://legacy.library.ucsf.edu/tid/qss 61f00/pdf;jessionid=AF356704CCB99DF3902ADD9 0B3EE4C31.tobacco03>
- [Online] Retrieved from the Internet: <URL:http://web.archive.org/web/20040811212 545/http://www.researchd.com/janssen/410200 .htm> [retrieved on 2005-08-01]

## Description

### Field of the invention

The present invention relates to new progesterone formulations for injectable use.

### State of the art

Progesterone (Pregn-4-ene-3,20-dione) presents numerous pharmacological applications. This hormone is used, for example, in assisted reproduction protocols, in the treatment of threatened miscarriage and for preventing habitual miscarriage.

In threatened miscarriage therapy, high plasma levels of progesterone, at least 200 nmol/ml, must be attained and maintained in order to achieve a therapeutic effect.

However, because of the slow gastrointestinal absorption and high hepatic metabolism of this hormone, such plasma levels of progesterone cannot be achieved by oral administration. Therefore, in the treatment of the aforesaid pathologies, progesterone is generally administered intramuscularly.

Because of the lipophilicity of progesterone, commercially available injectable formulations of this hormone are in the form of dispersions micronized in ethyl oleate. However, these types of formulations present a number of drawbacks: they are not well tolerated as they give rise to irritations at the injection site, they cannot be administered subcutaneously, this being the more easy and more danger-free route, and furthermore they make it necessary to administer high doses of progesterone, in the order of 100 mg per day, to obtain adequate plasma levels. On the other hand, solving said problems by administering aqueous solutions of synthetic or semisynthetic progestinic drugs has proved to be inadvisable, as these products often present adverse effects.

There is therefore a strongly felt need in the art for injectable aqueous solutions of progesterone.

It has been known for some time, for example from patents CN 1517091 US 4,727,064 and WO85/02767, that the water solubility of poorly water-soluble drugs such as progesterone can be increased by the formation of a complex with β-cyclodextrin ethers, specifically hydroxypropyl-β-cyclodextrin. In the aforesaid patents the suitability of these formulations for preparing injectable solutions is stated.

Although the aforesaid patents have now been granted for some years, there is currently no commercially available formulation for parenteral administration in the form of an aqueous solution comprising complexes of hydroxypropyl-β-cyclodextrin with progesterone. This suggests a considerable difficulty in the practical implementation of these types of formulations conceived on paper, made further apparent by the considerable need felt in the art.

The present inventors have prepared aqueous solutions of the hydroxypropyl-β-cyclodextrin-progesterone complex by following the teachings of the known art and have in fact found that these solutions are not stable over time and are therefore unusable by injection.

Specifically, if the solutions obtained after dissolving the complex are left to stand at ambient temperature, they form a precipitate after a period of about 24 hours. Even if the precipitate is removed by filtration, a precipitate re-forms anyway in the clear solution thus obtained after about 48 hours.

This phenomenon is not described in any of the aforementioned patents.

The precipitate does not dissolve in water, 96% EtOH, aqueous solutions containing propylene glycol or polysorbates such as Tween 20. The problem observed therefore makes it totally impossible to use the formulations described in the known art by means of injection.

### Summary of the invention

The present inventors have now surprisingly found that the formation of a precipitate from solutions of progesterone-hydroxypropyl-β-cyclodextrin complexes is related to the presence of unsubstituted β-cyclodextrin impurities in commercially available hydroxypropyl-β-cyclodextrin preparations for pharmaceutical use. The observed precipitation is surprising and unexpected particularly in the light of the water solubility of unsubstituted β-cyclodextrin which the known art states to be 1.8 g/100 ml, and of the small quantities thereof present in preparations of hydroxypropyl-β-cyclodextrin (usually not greater than 1 %).

The present inventors have also found that precipitate formation can be avoided if solutions of the hydroxypropyl-β-cyclodextrin-progesterone complex containing a quantity of unsubstituted β-cyclodextrin, less than 0.1 %, are prepared. These solutions are actually stable for at least 40 days at 25°C and are suitable for use by injection.

### Detailed description of the invention

Therefore, a first aspect of the present invention is the provision of injectable progesterone formulations comprising the hydroxypropyl-β-cyclodextrin-progesterone complex characterised by containing a quantity of unsubstituted β -cyclodextrin less than 0.1 % w/w on the quantity of hydroxypropyl-β-cyclodextrin.

The aforesaid formulations can be in the form of a ready-to-use aqueous solution or lyophilizate which is reconstituted in water when required to provide an extemporaneous injectable aqueous solution.

As will be demonstrated in examples 1,2,3 and 5 to follow, the aqueous solutions of the present invention are stable over time, without any precipitate forming for at least 40 days at 25°C and at least 48 hours at 5°C, being therefore suitable for use by injection.

Moreover, the solutions of the present invention can also contain very high concentrations of progesterone.

Therefore, the formulations of the present invention are particularly suitable for intramuscular and especially subcutaneous use, where small amounts of solution have to be used.

Hydroxypropyl-β-cyclodextrin, obtained by the propylation of β-cydodextrin hydroxyls, is commercially available with various degrees of substitution which indicate the average number of hydroxypropyl groups per cyclodextrin.

Commercial hydroxypropyl-β-cyclodextrin preparations contain impurities consisting of unsubstituted β-cyclodextrin whose quantity usually increases with decreasing degree of substitution of hydroxypropyl-β-cyclodextrin.

In accordance with a first embodiment, the formulations of the present invention can be obtained by preparing, following the methods already known to experts of the art, progesterone-hydroxypropyl-β-cyclodextrin complexes from preparations of hydroxypropyl-β-cyclodextrin containing a quantity of unmodified β-cyclodextrin less than 0.1 % on the quantity of hydroxypropylated-β-cyclodextrin as described in example 3.

However, all the hydroxypropyl-β-cyclodextrin preparations that are listed in the Pharmacopoeia and hence suitable for pharmaceutical use contain unsubstituted β -cyclodextrin impurities in quantities greater than the aforesaid values.

Therefore, in accordance with an alternative and particularly preferred embodiment, the formulations of the present invention can also be obtained starting from hydroxypropyl-β-cyclodextrin preparations that contain quantities of unsubstituted β-cyclodextrin greater than the aforesaid values, by means of a particular process established by the present inventors which enables the β -cyclodextrin quantity to be decreased to less than 0.1 % w/w on the quantity of hydroxypropylated β-cyclodextrin.

This process comprises the following steps:
a) preparing an aqueous solution of hydroxypropyl-β-cyclodextrin said hydroxypropyl-β-cyclodextrin containing a quantity of unsubstituted β-cyclodextrin between 0.3% and 1% w/w on the quantity of hydroxypropyl-β-cyclodextrin, and said solution having a concentration between 12% and 55% w/w and preferably 48% w/w;
b) adding micronized progesterone under stirring to the solution of step a), preferably in a quantity equal to 10% by weight on the quantity of hydroxypropyl-β-cyclodextrin, corresponding to the saturation concentration of the solution, and optionally filtering, preferably with a 0.45 µm filter, to obtain a transparent colourless solution;
c) maintaining the solution at a temperature between 2 and 8°C, preferably 5°C, for a period of at least 2 days, preferably between 2 and 10 days, preferably between 3 and 8 days and even more preferably 3 days, to hence obtain a precipitate;
d) carrying out a clarification filtration using a filter with a porosity between 0.45 and 0.8 micron.

Usually, in order to achieve complete dissolution of progesterone, micronized progesterone needs to be used in step b), and/or the mixture needs to be maintained under stirring for at least 30 minutes.

If injectable formulations in the form of ready-to-use solutions are to be prepared in step a) of the aforesaid procedure, water for injectable preparations is used; after step d) an additional step, e), is undertaken in which the solution obtained in step d) is sterilized by filtering through a 0.22 µm filter.

If instead injectable formulations in the form of lyophilizates to be reconstituted in water on use are to be prepared, the process of the invention comprises a step, e), in which the solution obtained in step d) is sterilized by filtering through a 0.22 µm filter followed by a step, f), in which the solution obtained in step e) is subjected to lyophilization in a sterile environment.

Before undertaking the lyophilization, the solution is preferably diluted until a progesterone-hydroxypropyl-β-cyclodextrin complex is obtained whose concentration is less than 27% w/w. As will be shown in examples 4 and 5 to follow, the present inventors have in fact found that by lyophilizing solutions at higher concentrations, powders are obtained which dissolve in water over very long periods of time, in the order of 30-40 minutes, while from the solution diluted in accordance with the invention a product is obtained which can easily be reconstituted in water, resulting in a clear solution being obtained in 5-10 minutes.

The present inventors have surprisingly found that lyophilization treatment also enables stability in water of the progesterone-hydroxypropyl-β-cyclodextrin complex to be further increased.

The present invention also relates to unit dosage forms for intramuscular or subcutaneous administration of progesterone consisting of formulations in accordance with the present invention containing a quantity of progesterone between 25 and 100 mg.

A further advantage of the formulations of the invention is that the injectable solutions of the present invention enable concentrations of plasma progesterone to be achieved that are 3 times greater than those achieved with oil dispersions of the known art for the same dosage, thus enabling the dosages of progesterone required to achieve effective plasma concentrations to be considerably reduced.

Moreover, as indicated in example 6, the present inventors have also found that lyophilization alone, when undertaken in the absence of the maturation process, also results in an increase in stability in water of the progesterone-hydroxypropyl-β-cyclodextrin complex. As already noted by the present inventors, in preparing lyophilizates of matured solutions, in order to obtain a lyophilizate which can be reconstituted in water within adequate time periods, lyophilization must be carried out on a solution in which the concentration of the progesterone-hydroxypropyl-β-cyclodextrin complex is less than 27% w/w.

The present invention will be better illustrated by the experimental examples which follow.

### Example 1

48 g of hydroxypropyl- β-cyclodextrin (degree of substitution equal to 0.63; 0.5% unsubstituted β cyclodextrin content) are dissolved, under magnetic stirring, in about 47 g of deionised water. 4.8 g of progesterone are added to the transparent colourless solution under magnetic stirring, and the final weight is made up to 100 g with deionised water. It is left under stirring for about 40 minutes. The solution is filtered through a 0.45 micron filter to obtain a transparent colourless solution.

The progesterone titre, effected by UV analysis, is found to be 55.52 mg/ml.

The solution thus obtained is maintained at 5°C for 8 days. Under these conditions a white precipitate forms which is removed from the solution by filtering through a 0.45 micron filter.

A transparent colourless solution is obtained, found to be stable at 25°C for at least 40 days.

The progesterone titre, effected by UV analysis, is found to be 52.54 mg/ml.

### Example 2

48 g of hydroxypropyl-β-cyclodextrin (degree of substitution equal to 0.59; 0.6% unsubstituted β-cyclodextrin content) are dissolved, under magnetic stirring, in about 47 g of deionised water. 4.8 g of progesterone are added to the transparent colourless solution under magnetic stirring, and the final weight is made up to 100 g with deionised water. It is left under stirring for about 40 minutes. The solution is filtered through a 0.45 micron filter to obtain a transparent colourless solution.

The progesterone titre, effected by UV analysis, is found to be 54.44 mg/ml.

The solution thus obtained is maintained at 5°C for 3 days. Under these conditions a white precipitate forms which is removed from the solution by filtering through a 0.45 micron filter.

A transparent colourless solution is obtained, found to be stable at 25°C for at least 40 days.

The progesterone titre, effected by UV analysis, is found to be 51.37 mg/ml.

### Example 3

3 solutions are prepared in parallel by dissolving in 47.2 g of deionised water under magnetic stirring, for each of these solutions, 48 g of hydroxypropyl-β-cyclodextrin containing respectively 0.6% (sol. 1), 0.5% (sol. 2) and less than 0.1 % (sol. 3) of unsubstituted β-cyctodextrin. When the solutions are transparent and colourless 4.8 g of progesterone are added and are then left under stirring for about 40 minutes.

The solutions obtained are clarified through a 0.45 micron filter to obtain a solution free of solid bodies. The clear solutions are maintained at a temperature of 5°C for 15 days. Under these conditions a white precipitate forms after 1 day for solution 1 and after 4 days for solution 2 giving rise to solution cloudiness whereas, in the case of solution 3, no precipitate or cloudiness is found. Solutions 1 and 2 are filtered through a 0.45 micron filter after maturing for 15 days at 5°C.

All three solutions obtained are transparent and colourless and are stable at 25°C for at least 40 days.

### Example 4

48 g of hydroxypropyl-β-cyclodextrin (degree of substitution 0.63, 0.5% unsubstituted β-cyclodextrin content) are dissolved, under magnetic stirring, in 47.2 g of deionised water. When the solution is transparent and colourless, 4.8 g of progesterone are added and left under stirring for about 40 minutes.

The solution obtained is clarified through a 0.45 micron filter to obtain a solution free of solid bodies. The clear solution is maintained at a temperature of 4-6°C for 8 days. Under these conditions a white precipitate forms which is removed from the solution by further filtration with a 0.45 micron filter.

The solution thus obtained is dispensed in vials to the extent of 1.1 g/vial and then, after stoppering, placed in the lyophilizer chamber and subjected to the lyophilization process. The lyophilizate obtained is compact and ivory-white in colour.

The sample thus obtained is treated with 1 ml of water for injection and forms a clear solution after 30 minutes without applying any stirring.

The progesterone titre, effected by UV analysis on the reconstituted product, is found to be 32.75 mg/g, equal to 49 mg/vial.

### Example 5

Two 40 g samples of a matured solution, prepared as described in example 4, are diluted respectively with 60 g (Sol. A, dilution 1 → 2.5) and 40 g (Sol B, dilution 1 → 2) of deionised water maintaining the solution under magnetic stirring for 10 minutes.

The two solutions thus prepared are split in parallel into vials to the extent of 2.5 g/vial and of 2 g/vial. After stoppering, the vials are inserted into the lyophilizer chamber and subjected to the lyophilization process, to obtain ivory-white coloured compact lyophilizates.

The lyophilizates obtained are reconstituted utilizing 1 ml of water for injection per vial. The time needed for complete dissolution, with the lyophilizates obtained from both diluted solutions, was found to be under 10 minutes.

The progesterone titre, effected by UV analysis on the solutions prior to lyophilization, was found to be 46 mg/vial in the case of solution A and 46.5 mg/vial in the case of solution B. The UV analyses on the reconstituted product confirm the concentrations of both samples.

The lyophilizate, following reconstitution with 1 ml of water for injection, is found to be clear and colourless without precipitate formation for at least 50 days at 25°C and at least 5 days at 5°C.

The results obtained demonstrate that the dilution of the solution prior to lyophilization provides a considerable reduction in the time needed for lyophilizate reconstitution.

### Example 6

6a) A solution of progesterone and hydroxypropyl-β-cyclodextrin is prepared as described in example 4. However, instead of maintaining the solution at low temperature for 8 days, vial filling and lyophilization are carried out directly as described in example 4.
   The lyophilizate obtained is compact and ivory-white in colour.
   The sample thus obtained, treated with 1 ml of water for injection, forms a clear solution after 30 minutes without applying any stirring.
   The progesterone titre, effected by UV analysis on the reconstituted product, is found to be 34.7 mg/g, equal to 52 mg/vial.
6b) A solution of progesterone and hydroxypropyl-β-cyclodextrin is prepared as described in example 4, but omitting the maturation stage. 40 g of the non matured solution is diluted with 60 g of water and vial filling and lyophilization are carried out directly, as described in example 4.

The lyophilizate obtained is compact and ivory-white in colour.

It is reconstituted using 1 ml of water for injection.

The time needed for complete dissolution of the buffer was found to be under 10 minutes.

The progesterone titre, achieved by UV on the solution prior to lyophilization, is found to be 47.3 mg/2.5 g of solution and the lyophilizate concentration, reconstituted with 1 ml of water for injection, is found to be 30.9 mg/g equal to 46.3 mg/vial.

After reconstituting with 1 ml of water the lyophilizate is found to be clear and colourless without a precipitate forming for at least 20 days at 25°C and at least 5 days at 5°C. After this period the formation of a precipitate is observed which in the space of 24 hours adheres to the base of the vial.

The result obtained in example 6b shows that direct lyophilization of hydroxypropyl-β-cyclodextrin solutions, even in the absence of the solution maturation process, leads to formulations being obtained which, once reconstituted in water, give rise to solutions with improved stability compared with the initial solutions.

## Claims

1. Injectable progesterone formulation comprising a complex between progesterone and hydroxypropyl-β-cyclodextrin, **characterised by** containing a quantity of unsubstituted β-cyclodextrin less than 0.1% w/w on the quantity of hydroxypropyl-β-cyclodextrin.

2. Formulation as claimed in claim 1 suitable for intramuscular or subcutaneous use.

3. Formulation as claimed in claims 1 to 2 in the form of a ready-to-use aqueous solution.

4. Formulation as claimed in claims 1 to 2 in the form of a lyophilizate for the extemporaneous preparation of an injectable aqueous solution.

5. Process for preparing a formulation as claimed in claims 1 to 4 comprising the following steps:
a) preparing an aqueous solution of hydroxypropyl-β-cyclodextrin said hydroxypropyl-β-cyclodextrin containing a quantity of unsubstituted β-cyclodextrin between 0.3% and 1% w/w on the quantity of hydroxypropyl-β-cyclodextrin, and said solution having a concentration between 12% and 55% w/w;
b) adding progesterone under stirring to the solution of step a) and optionally filtering to obtain a transparent colourless solution;
c) maintaining the solution at a temperature between 2 and 8°C for a period of at least 2 days;
d) carrying out a clarification filtration using a filter with a porosity between 0.45 and 0.8 micron.

6. Process as claimed in claim 5 wherein in step a) the hydroxypropyl-β-cyclodextrin concentration is 48% w/w.

7. Process as claimed in claim 5 or 6 wherein in step b) the progesterone is added in a quantity equal to 10% by weight on the quantity of hydroxypropyl-β-cyclodextrin.

8. Process as claimed in claims 5 to 7 wherein in step b) the progesterone is added in micronized form.

9. Process as claimed in claims 5 to 8 wherein in step c) the solution is maintained at a temperature of 2-8°C for a period between 2 and 10 days.

10. Process as claimed in claim 9 wherein in step c) the solution is maintained at a temperature of 2-8°C for a period between 3 and 8 days.

11. Process as claimed in claims 5 to 10 wherein in step c) the solution is temperature controlled at 5°C.

12. Process as claimed in claims 5 to 11 wherein said formulation is in the form of a ready-to-use aqueous solution, water for injectable preparations is used in step a) and, after step d), a further step e) is undertaken in which the solution of step d). is sterilized by filtering through a 0.22 µm filter.

13. Process as claimed in claims 5 to 11 wherein said formulation is in the form of a lyophilizate for the extemporaneous preparation of an injectable solution and after step d) a step e) is undertaken in which the solution of step d) is subjected to sterilization by filtering through a 0.22 µm filter followed by a step f) in which the solution is lyophilized in a sterile environment.

14. Process as claimed in claim 13 wherein in said step f), before carrying out the lyophilization, the solution obtained in step e) is diluted with water until a concentration less than 27% w/w of the progesterone-hydroxypropyl-β-cyclodextrin complex is obtained.

15. A unit dosage form for intramuscular or subcutaneous progesterone administration consisting of a formulation as claimed in claims 1 to 4 containing progesterone in a quantity between 25 and 100 mg.

## Patentansprüche

1. Injizierbare Progesteronformulierung umfassend einen Komplex zwischen Progesteron und Hydroxypropyl-β-cyclodextrin, **dadurch gekennzeichnet, dass** unsubstituiertes β-Cyclodextrin in einer Menge von weniger als 0,1 Gewichtsprozent (Gew.-%) der Hydroxypropyl-β-cyclodextrin-Menge enthalten ist.

2. Formulierung, wie in Anspruch 1 beansprucht, geeignet zur intramuskulären oder subkutanen Verwendung.

3. Formulierung, wie in den Ansprüchen 1 bis 2 beansprucht, in Form einer gebrauchsfertigen wässrigen Lösung.

4. Formulierung, wie in den Ansprüchen 1 bis 2 beansprucht, in Form eines Lyophilisats zur Magistralrezeptur (*extemporaneous preparation*) einer injizierbaren wässrigen Lösung.

5. Verfahren zur Herstellung einer Formulierung, wie in den Ansprüchen 1 bis 4 beansprucht, umfassend die folgenden Schritte:
(a) Herstellen einer wässrigen Lösung von Hydroxypropyl-β-cyclodextrin, wobei das Hydroxypropyl-β-cyclodextrin unsubstituiertes β-Cyclodextrin in einer Menge zwischen 0,3 Gew.-% und 1 Gew.-% der Hydroxypropyl-β-cyclodextrin-Menge enthält und die Lösung eine Konzentration von zwischen 12 Gew.-% und 55 Gew.-% aufweist;
(b) Zugeben von Progesteron zur Lösung aus Schritt a) unter Rühren und wahlweise Abfiltrieren, um eine transparente, farblose Lösung zu erhalten;
(c) Halten der Lösung bei einer Temperatur zwischen 2 und 8°C für einen Zeitraum von mindestens 2 Tagen;
(d) Durchführen einer Klärungsfiltration unter Verwendung eines Filters mit einer Porengröße von zwischen 0,45 und 0,8 Mikrometern.

6. Verfahren, wie in Anspruch 5 beansprucht, wobei in Schritt a) die Hydroxypropyl-β-cyclodextrin-Konzentration 48 Gew.-% ist.

7. Verfahren, wie in Anspruch 5 oder 6 beansprucht, wobei in Schritt b) das Progesteron in einer Menge entsprechend 10 Gew.-% der Hydroxypropyl-β-cyclodextrin-Menge zugegeben wird.

8. Verfahren, wie in den Ansprüchen 5 bis 7 beansprucht, wobei in Schritt b) das Progesteron in mikronisierter Form zugegeben wird.

9. Verfahren, wie in den Ansprüchen 5 bis 8 beansprucht, wobei in Schritt c) die Lösung bei einer Temperatur von 2-8°C für einen Zeitraum von zwischen 2 und 10 Tagen gehalten wird.

10. Verfahren, wie in Anspruch 9 beansprucht, wobei in Schritt c) die Lösung bei einer Temperatur von 2-8°C für einen Zeitraum von zwischen 3 und 8 Tagen gehalten wird.

11. Verfahren, wie in den Ansprüchen 5 bis 10 beansprucht, wobei in Schritt c) die Lösung bei 5°C temperaturgesteuert wird.

12. Verfahren, wie in den Ansprüchen 5 bis 11 beansprucht, wobei die Formulierung in Form einer gebrauchsfertigen Lösung vorliegt, in Schritt a) Wasser für die injizierbaren Zubereitungen verwendet wird, und nach Schritt d) ein weiterer Schritt e) vorgenommen wird, in dem die Lösung aus Schritt d) mittels Filtrierung durch einen 0,22 µm-Filter sterilisiert wird.

13. Verfahren, wie in den Ansprüchen 5 bis 11 beansprucht, wobei die Formulierung in Form eines Lyophilisats zur Magistralrezeptur einer injizierbaren Lösung vorliegt und nach Schritt d) ein Schritt e) vorgenommen wird, in dem die Lösung aus Schritt d) der Sterilisation mittels Filtrierung durch einen 0,22 µm-Filter unterzogen wird, gefolgt von einem Schritt f), in dem die Lösung in einer sterilen Umgebung lyophilisiert wird.

14. Verfahren, wie in Anspruch 13 beansprucht, wobei in Schritt f), bevor die Lyophilisation durchgeführt wird, die in Schritt e) erhaltene Lösung mit Wasser verdünnt wird, bis eine Konzentration von weniger als 27 Gew.-% w/w des Progesteron-Hydroxypropyl-β-cyclodextrin-Komplexes erhalten wird.

15. Dosierungseinheit zur intramuskulären oder subkutanen Progesteronverabreichung, bestehend aus einer Formulierung, wie in den Ansprüchen 1 bis 4 beansprucht, enthaltend Progesteron in einer Menge von zwischen 25 und 100 mg.

## Revendications

1. Formulation injectable à base de progestérone comprenant un complexe entre la progestérone et l'hydroxypropyl-β-cyclodextrine, **caractérisée par le fait qu'**elle contient une quantité de β-cyclodextrine non substituée inférieure à 0,1 % en p/p par rapport à la quantité d'hydroxypropyl-β-cyclodextrine.

2. Formulation selon la revendication 1 adaptée à une administration par voie intramusculaire ou sous-cutanée.

3. Formulation selon les revendications 1 à 2 sous la forme d'une solution aqueuse prête à l'emploi.

4. Formulation selon les revendications 1 à 2 sous la forme d'un lyophilisat pour la préparation extemporanée d'une solution aqueuse injectable.

5. Procédé de préparation d'une formulation selon les revendications 1 à 4 comprenant les étapes suivantes :
a) préparation d'une solution aqueuse d'hydroxypropyl-β-cyclodextrine, ladite hydroxypropyl-β-cyclodextrine contenant une quantité de β-cyclodextrine non substituée comprise entre 0,3 % et 1 % en p/p par rapport à la quantité d'hydroxypropyl-β-cyclodextrine et ladite solution présentant une concentration comprise entre 12 % et 55 % en p/p ;
b) ajout de progestérone sous agitation à la solution de l'étape a) et facultativement filtration pour obtenir une solution incolore transparente ;
c) maintien de la solution à une température comprise entre 2 et 8 °C pendant une durée d'au moins 2 jours ;
d) réalisation d'une filtration de clarification à l'aide d'un filtre dont la porosité est comprise entre 0,45 et 0,8 micron.

6. Procédé selon la revendication 5 dans lequel, lors de l'étape a), la concentration de l'hydroxypropyl-β-cyclodextrine est de 48 % en p/p.

7. Procédé selon la revendication 5 ou 6 dans lequel, lors de l'étape b), la progestérone est ajoutée en une quantité égale à 10 % en poids par rapport à la quantité d'hydroxypropyl-β-cyclodextrine.

8. Procédé selon les revendications 5 à 7 dans lequel, lors de l'étape b), la progestérone est ajoutée sous forme micronisée.

9. Procédé selon les revendications 5 à 8 dans lequel, lors de l'étape c), la solution est maintenue à une température allant de 2 à 8 °C pendant une durée comprise entre 2 et 10 jours.

10. Procédé selon la revendication 9 dans lequel, lors de l'étape c), la solution est maintenue à une température allant de 2 à 8 °C pendant une durée comprise entre 3 et 8 jours.

11. Procédé selon les revendications 5 à 10 dans lequel, lors de l'étape c), la solution est fixée à une température de 5 °C.

12. Procédé selon les revendications 5 à 11 dans lequel ladite formulation se présente sous la forme d'une solution aqueuse prête à l'emploi, de l'eau pour préparations injectables est utilisée à l'étape a) et, après l'étape d), une étape e) supplémentaire est réalisée, dans laquelle la solution de l'étape d) est stérilisée par filtration à travers un filtre de 0,22 µm.

13. Procédé selon les revendications 5 à 11 dans lequel ladite formulation se présente sous la forme d'un lyophilisat pour la préparation extemporanée d'une solution injectable et après l'étape d), une étape e) est réalisée dans laquelle la solution de l'étape d) est soumise à une stérilisation par filtration à travers un filtre de 0,22 µm, suivie d'une étape f) dans laquelle la solution est lyophilisée dans un environnement stérile.

14. Procédé selon la revendication 13 dans lequel, lors de ladite étape f), avant de réaliser la lyophilisation, la solution obtenue à l'étape e) est diluée avec de l'eau jusqu'à ce obtention d'une concentration inférieure à 27 % en p/p du complexe progestérone-hydroxypropyl-β-cyclodextrine.

15. Forme galénique unitaire destinée à une administration par voie intramusculaire ou sous-cutanée de progestérone se composant d'une formulation selon les revendications 1 à 4 contenant de la progestérone en une quantité comprise entre 25 et 100 mg.
